# EUROPEAN PATENT SPECIFICATION

(11) **EP 1 807 041 B1**
(45) Date of publication and mention of the grant of the patent: **02.01.2008**
(21) Application number: 05796902.4
(22) Date of filing: 07.10.2005
(51) Int. Cl.: A61K 6/00

(54) **PERSONAL CARE COMPOSITIONS WITH SALTS OF HYDROXYPROPYL TRIALKYLAMMONIUM SUBSTITUTED MONO-SACCHARIDE**
KÖRPERPFLEGEZUSAMMENSETZUNGEN MIT SALZEN AUS HYDROXYPROPYL-TRIALKYLAMMONIUM-SUBSTITUIERTEN MONOSACCHARIDEN
COMPOSITIONS D'HYGIENE PERSONNELLE A SELS DE MONOSACCHARIDE A SUBSTITUTION HYDROXYPROPYL TRIALKYLAMMONIUM

(30) Priority: 25.10.2004 US 972590
(43) Date of publication of application: 18.07.2007
(73) Proprietor: UNILEVER PLC, London, Greater London EC4P 4BQ (GB); UNILEVER N.V., 3013 AL Rotterdam (NL)
(72) Inventor: MCMANUS, Richard L., Unilever H. & P. Care USA, Trumbull, Connecticut 06611 (US); SPADINI, Alessandro Luigi, Stamford, Connecticut 06907 (US); CHENEY, Michael C., Unilever H. & P. Care USA, Trumbull, Connecticut 0611 (US); MINER, Philip Edward, Unilever H. & P. Care USA, Trumbull, Connecticut 06611 (US)
(74) Representative: Acham, Nicholas Clive
(86) International application number: PCT/EP2005/010906
(87) International publication number: WO 2006/045428

(56) References cited:
- US-A1- 2003 095 990
- US-A1- 2004 022 818
- US-A1- 2004 110 651

## Description

### Technical Field of the Invention

The invention concerns personal care compositions providing moisturization both in high and low relative humidity environments.

### Background of the Invention

Dry skin is a problem in varying degree to most humans. This condition is particularly evident in winter. Personal care products such as skin creams/lotions, shampoos/conditioners, toilette bars/shower gels and antiperspirant/deodorants are normally formulated with at least one material to address dry skin. Symptoms such as itching, flaking and a visually displeasing dermal appearance can all to some extent be modulated.

There are three classes of materials employed against the problem. Occlusives such as petrolatum or silicone oils serve to inhibit loss of natural moisture. They form a barrier between the epidermis and the environment. Another approach is the use of keratolytic agents to enhance the rate of dermal exfoliation. Alpha-hydroxy acids are the most common agents for achieving exfoliation.

A third approach to dry skin is topical application of humectants. Hydroxylated monomeric and polymeric organic substances are generally used for this purpose. Glycerin known also as glycerol is one of the most effective humectants.

There are several shortcomings in the performance of known humectants. Even the best such as glycerin requires to be formulated at' relatively high levels to achieve good moisturization. Secondly, known humectants perform well in high relative humidity environments; however, hardly any of these substances provide effectiveness at low relative humidity (i.e. less than 20% moisture at 20°C). Average indoor relative humidity during winter is approximately 13% in areas such as the north-east U.S. It is quite evident that a real need exists for an improved moisturization technology.

A moisturizer known as Honeyquat 50 with INCI name of Hydroxypropyltrimonium Honey has been reported to be a better humectant than glycerin (see the Arch/Brooks brochure titled "Cosmetic Ingredients & Ideas®", Issue No. 2, August 2001). Honeyquat 50 is described as being derived from the reaction of pendent hydroxyl groups (on the disaccharide) of a "light" deodorized grade of honey with a chlorohydroxytrimethylammonium derivative. Although this substance has excellent humectancy, moisturization at low relative humidity still remains to be conquered.

Accordingly, the present invention seeks to identify humectants which are operative not only at high but also low relative humidity, for application in personal care products.

### Summary of the Invention

A personal care composition is provided which includes:
(i) from 0.1 to 30% by weight of a quaternary ammonium (quat) compound which is a salt of hydroxypropyltri(C₁-C₃ alkyl)ammonium monosubstituted monosaccharide; and
(ii)a cosmetically acceptable carrier.

### Detailed Description of the Invention

Now it has been found that salts of hydroxypropyltri(C₁-C₃)ammonium monosubstituted monosaccharides are excellent humectants under both high and low relative humidity conditions. Amounts of these salts may range from 0.1 to 30%, preferably from 0.5 to 25%, more preferably from 1 to 20%, and optimally from 1.5 to 12% by weight of the composition.

Salts of hydroxypropyltri(C₁-C₃ alkyl) ammonium mono-substituted monosaccharides can be formed in a variety of procedures. Most preferred is via reaction of 2-hydroxy-3-chloropropyl trimethylammonium chloride with a monosaccharide in an approximately 1:1 molar ratio in an alkaline medium. By typical Williamson synthesis, sodium chloride is eliminated thereby forming an ether linkage between the hydroxypropyl end of the quat group and the mono-saccharide.

Monosaccharides, particularly reducing and non-reducing cyclic monosaccharides, are the smallest carbohydrate molecules encompassing the four-, five- and six- carbon sugars. Illustrative monosacchrides are ribose, deoxyribose, glucose, fructose, arabinose, xylose, lyxose, allose, altrose, gulose, mannose, idose, galactose and talose. Most preferred are glucose and fructose as the monosaccharide moiety which is to be substituted with the hydroxypropyltrimonium group.

Ordinarily the C₁-C₃ alkyl substituent on the quaternized ammonium group will be methyl, ethyl, n-propyl, isopropyl or hydroxyethyl and mixtures thereof. Particularly preferred is a trimethyl ammonium group known through INCI nomenclature as a "trimonium" group. Any anion can be used in the quat salt. The anion may be organic or inorganic with the proviso that the material is cosmetically acceptable. Typical inorganic anions are halides, sulfates, phosphates, nitrates and borates. Most preferred are the halides, especially chloride. Organic anionic counter ions include methosulfate, toluoyl sulfate, acetate, citrate, tartrate, glycolate, lactate, gluconate, and benzenesulfonate.

Particularly preferred quaternary ammonium salts of the present invention are illustrated by structures I and II below, wherein X⁻ is a halide. These formulas are intended as including all comformational isomers of the depicted structures.

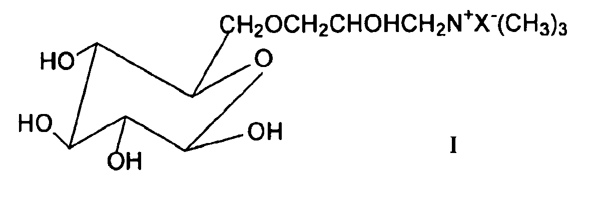

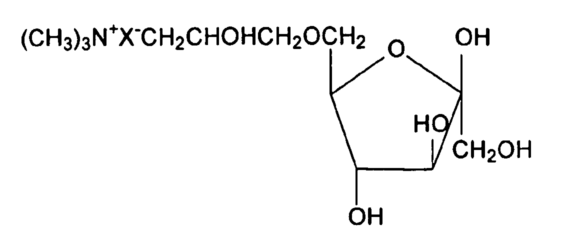

Advantageously, compositions of the present invention will be formulated with a quaternary ammonium salt where the monosaccharide is only mono-substituted with hydroxypropyltri(C₁-C₃ alkyl) ammonium groups. However, smaller amounts of di- and tri- substituted monosaccharide may also be present. These amounts normally may range from 0 to 20%, possibly from 2 to 10% by weight based on the weight of the quaternary ammonium compound present. More specifically, the multi-substituted monosaccharide may be di-[hydroxypropyltri(C₁-C₃ alkyl) ammonium] monosaccharide, tri-[hydroxypropyltri(C₁-C₃ alkyl) ammonium] monosaccharide and mixtures thereof.

By the term personal care composition is meant any substance applied to a human body for improving appearance, cleansing, odor control or general aesthetics. Non-limiting examples of personal care compositions include leave-on skin lotions and creams, shampoos, hair conditioners, shower gels, toilet bars, antiperspirants, deodorants, dental products, shave creams, depilatories, lipsticks, foundations, mascara, sunless tanners and sunscreen lotions.

Compositions of this invention will also include a cosmetically acceptable carrier. Amounts of the carrier may range from 1 to 99.9%, preferably from 70 to 95%, optimally from 80 to 90% by weight of the composition. Among the useful carriers are water, emollients, fatty acids, fatty alcohols, thickeners and combinations thereof. The carrier may be aqueous, anhydrous or an emulsion. Preferably the compositions are aqueous, especially water and oil emulsions of the W/O or O/W or triplex W/O/W variety. Water when present may be in amounts ranging from 5 to 95%, preferably from 20 to 70%, optimally from 35 to 60% by weight of the composition.

Emollient materials may serve as cosmetically acceptable carriers. These may be in the form of silicone oils, natural or synthetic esters and hydrocarbons. Amounts of the emollients may range anywhere from 0.1 to 95%, preferably between 1 and 50% by weight of the composition.

Silicone oils may be divided into the volatile and non-volatile variety. The term "volatile" as used herein refers to those materials which have a measurable vapor pressure at ambient temperature. Volatile silicone oils are preferably chosen from cyclic (cyclomethicone) or linear polydimethylsiloxanes containing from 3 to 9, preferably from 4 to 5, silicon atoms.

Non-volatile silicone oils useful as an emollient material include polyalkyl siloxanes, polyalkylaryl siloxanes and polyether siloxane copolymers. The essentially non-volatile polyalkyl siloxanes useful herein include, for example, polydimethyl siloxanes with viscosities of from 5 x 10⁻⁶ to 0.1 m²/s at 25°C. Among the preferred non-volatile emollients useful in the present compositions are the polydimethyl siloxanes having viscosities from 1 x 10⁻⁵ to 4 x 10⁻⁴ m²/s at 25°C.

Another class of non-volatile silicones are emulsifying and non-emulsifying silicone elastomers. Representative of this category is dimethicone/vinyl dimethicone crosspolymer available as Dow Corning 9040, General Electric SFE 839, and Shin-Etsu KSG-18. Silicone waxes such as Silwax WS-L (dimethicone copolyol laurate) may also be useful.

Among the ester emollients are:
a) Alkyl esters of saturated fatty acids having 10 to 24 carbon atoms. Examples thereof include behenyl neopentanoate, isononyl isonanonoate, isopropyl myristate and octyl stearate.
b) Ether-esters such as fatty acid esters of ethoxylated saturated fatty alcohols.
c) Polyhydric alcohol esters such as ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monostearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxyethylene sorbitan fatty acid esters. Particularly useful are pentaerythritol, trimethylolpropane and neopentyl glycol esters of C₁-C₃₀ alcohols.
d) Wax esters such as beeswax, spermaceti wax and tribehenin wax.
e) Sugar ester of fatty acids such as sucrose polybehenate and sucrose polycottonseedate.

Natural ester emollients principally are based upon mono-, di- and tri- glycerides. Representative glycerides include sunflower seed oil, cottonseed oil, borage oil, borage seed oil, primrose oil, castor and hydrogenated castor oils, rice bran oil, soybean oil, olive oil, safflower oil, shea butter, jojoba oil and combinations thereof. Animal derived emollients are represented by lanolin oil and lanolin derivatives. Amounts of the natural esters may range from 0.1 to 20% by weight of the composition.

Hydrocarbons which are suitable cosmetically acceptable carriers include petrolatum, mineral oil, C₁₁-C₁₃ isoparaffins, polybutenes and especially isohexadecane, available commercially as Permethyl 101A from Presperse Inc.

Fatty acids having from 10 to 30 carbon atoms may also be suitable as cosmetically acceptable carriers. Illustrative of this category are pelargonic, lauric, myristic, palmitic, stearic, isostearic, oleic, linoleic, linolenic, hydroxystearic and behenic acids.

Fatty alcohols having from 10 to 30 carbon atoms are another useful category of cosmetically acceptable carrier. Illustrative of this category are stearyl alcohol, lauryl alcohol, myristyl alcohol, oleyl alcohol and cetyl alcohol.

Thickeners can be utilized as part of the cosmetically acceptable carrier of compositions according to the present invention. Typical thickeners include crosslinked acrylates (e.g. Carbopol 982®), hydrophobically-modified acrylates (e.g. Carbopol 1382®), polyacrylamides (e.g. Sepigel 305®), acryloylmethylpropane sulfonic acid/salt polymers and copolymers (e.g. Aristoflex HMB® and AVC®), cellulosic derivatives and natural gums. Among useful cellulosic derivatives are sodium carboxymethylcellulose, hydroxypropyl methocellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums. Inorganics may also be utilized as thickeners, particularly clays such as bentonites and hectorites, fumed silicas, talc, calcium carbonate and silicates such as magnesium aluminum silicate (Veegum®). Amounts of the thickener may range from 0.0001 to 10%, usually from 0.001 to 1%, optimally from 0.01 to 0.5% by weight of the composition.

Adjunct humectants may be employed in the present invention. These are generally polyhydric alcohol-type materials. Typical polyhydric alcohols include glycerol, propylene glycol, dipropylene glycol, polypropylene glycol, polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. The amount of adjunct humectant may range anywhere from 0.5 to 50%, preferably between 1 and 15% by weight of the composition.

Personal care compositions of the present invention may be in any form. These forms may include lotions, creams, roll-on formulations, sticks, mousses, aerosol and non-aerosol sprays and fabric (e.g. non-woven textile)-applied formulations.

Surfactants may also be present in compositions of the present invention. Total concentration of the surfactant when present may range from 0.1 to 90%, preferably from 1 to 40%, optimally from 1 to 20% by weight of the composition, being highly dependent upon the type of personal care product. The surfactant may be selected from the group consisting of anionic, nonionic, cationic and amphoteric actives. Particularly preferred nonionic surfactants are those with a C₁₀-C₂₀ fatty alcohol or acid hydrophobe condensed with from 2 to 100 moles of ethylene oxide or propylene oxide per mole of hydrophobe; C₂-C₁₀ alkyl phenols condensed with from 2 to 20 moles of alkylene oxide; mono- and di-fatty acid esters of ethylene glycol; fatty acid monoglyceride; sorbitan, mono- and di- C₈-C₂₀ fatty acids; and polyoxyethylene sorbitan as well as combinations thereof. Alkyl polyglycosides and saccharide fatty amides (e.g. methyl gluconamides) and trialkylamine oxides are also suitable nonionic surfactants.

Useful amphoteric surfactants include cocoamidopropyl betaine, C₁₂-C₂₀ trialkyl betaines, sodium lauroamphoacetate, and sodium laurodiamphoacetate.

Preferred anionic surfactants include soap, alkyl ether sulfates and sulfonates, alkyl sulfates and sulfonates, alkylbenzene sulfonates, alkyl and dialkyl sulfosuccinates, C₈-C₂₀ acyl isethionates, C₈-C₂₀ alkyl ether phosphates, C₈-C₂₀ sarcosinates, C₈-C₂₀ acyl lactylates, sulfoacetates and combinations thereof.

Sunscreen agents may also be included in compositions of the present invention. Particularly preferred are such materials as ethylhexyl p-methoxycinnamate (Parsol Mix®), avobenzene (Parsol 1789®) and benzophenone-3 (also known as oxybenzone). Inorganic sunscreen actives may be employed such as microfine titanium dioxide and zinc oxide. Amounts of the sunscreen agents when present may generally range from 0.1 to 30%, preferably from 2 to 20%, optimally from 4 to 10% by weight of the composition.

Antiperspirants and deodorant compositions of the present invention ordinarily will contain astringent actives. Examples include aluminum chloride, aluminum chlorhydrex, aluminum-zirconium chlorhydrex glycine, aluminum sulfate, zinc sulfate, zirconium and aluminum chlorohydroglycinate, zirconium hydroxychloride, zirconium and aluminum lactate, zinc phenolsulfonate and combinations thereof. Amounts of the astringents may range anywhere from 0.5 to 50% by weight of the composition.

Dental products formulated according to the present invention will generally contain a fluoride source to prevent' dental caries. Typical anti-caries actives include sodium fluoride, stannous fluoride and sodium monofluoro phosphate. Amounts of these materials will be determined by the amount of fluoride releasable which should range between 500 to 8800 ppm of the composition. Other components of dentifrices can include desensitizing agents such as potassium nitrate and strontium nitrate, sweeteners such as sodium saccharine, aspartame, sucralose, and potassium acesulfam. Thickeners, opacifying agents, abrasives and colorants will normally also be present.

Preservatives can desirably be incorporated into the personal care compositions of this invention to protect against the growth of potentially harmful microorganisms. Particularly preferred preservatives are phenoxyethanol, methyl paraben, propyl paraben, imidazolidinyl urea, dimethyloldimethylhydantoin, ethylenediaminetetraacetic acid salts (EDTA), sodium dehydroacetate, methylchloroisothiazolinone, methylisothiazolinone, iodopropynbutylcarbamate and benzyl alcohol. The preservatives should be selected having regard for the use of the composition and possible incompatibilities between the preservatives and other ingredients. Preservatives are preferably employed in amounts ranging from 0.0001% to 2% by weight of the composition.

Compositions of the present invention may include vitamins. Illustrative vitamins are vitamin A (retinol), vitamin B₂, vitamin B₃ (niacinamide), vitamin B₆, vitamin C, vitamin E, folic acid and biotin. Derivatives of the vitamins may also be employed. For instance, vitamin C derivatives include ascorbyl tetraisopalmitate, magnesium ascorbyl phosphate and ascorbyl glycoside. Derivatives of vitamin E include tocopheryl acetate, tocopheryl palmitate and tocopheryl linoleate. DL-panthenol and derivatives may also be employed. Total amount of vitamins when present in compositions according to the present invention may range from 0.001 to 10%, preferably from 0.01% to 1%, optimally from 0.1 to 0.5% by weight of the composition.

Another type of useful substance can be that of an enzyme such as amylases, oxidases, proteases, lipases and combinations. Particularly preferred is superoxide dismutase, commercially available as Biocell SOD from the Brooks Company, USA.

Skin lightening compounds may be included in the compositions of the invention. Illustrative substances are placental extract, lactic acid, niacinamide, arbutin, kojic acid, ferulic acid, resorcinol and derivatives including 4-substituted resorcinols and combinations thereof.. Amounts of these agents may range from 0.1 to 10%, preferably from 0.5 to 2% by weight of the composition.

Desquamation promoters may be present. Illustrative are the alpha-hydroxycarboxylic acids and beta-hydroxycarboxylic acids. The term "acid" is meant to include not only the free acid but also salts and C₁-C₃₀ alkyl or aryl esters thereof and lactones generated from removal of water to form cyclic or linear lactone structures. Representative acids are glycolic, lactic and malic acids. Salicylic acid is representative of the beta-hydroxycarboxylic acids. Amounts of these materials when present may range from 0.01 to 15% by weight of the composition.

A variety of herbal extracts may optionally be included in compositions of this invention. The extracts may either be water soluble or water-insoluble carried in a solvent which respectively is hydrophilic or hydrophobic. Water and ethanol are the preferred extract solvents. Illustrative extracts include those from green tea, chamomile, licorice, aloe vera, grape seed, citrus unshui, willowbark, sage, thyme and rosemary.

Also included may be such materials as lipoic acid, retinoxytrimethylsilane (available from Clariant Corp. under the Silcare 1M-75 trademark), dehydroepiandrosterone (DHEA) and combinations thereof. Ceramides (including Ceramide 1, Ceramide 3, Ceramide 3B and Ceramide 6) as well as pseudoceramides may also be useful. Amounts of these materials may range from 0.000001 to 10%, preferably from 0.0001 to 1% by weight of the composition.

Colorants, opacifiers and abrasives may also be included in compositions of the present invention. Each of these substances may range from 0.05 to 5%, preferably between 0.1 and 3% by weight of the composition.

The compositions of the present invention can also be, optionally, incorporated into a water insoluble substrate for application to the skin such as in the form of a treated wipe.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

Except in the operating and comparative examples, or where otherwise explicitly indicated, all numbers in this description indicating amounts of material may also be understood as modified by the word "about".

The following examples will more fully illustrate the embodiments of this invention. All parts, percentages and proportions referred to herein and in the appended claims are by weight unless otherwise illustrated.

### EXAMPLE 1

A representative personal care composition of the present invention in the form of a cosmetic lotion is outlined under Table I.

**TABLE I**

| INGREDIENT | WEIGHT % |
|---|---|
| PHASE A | |
| Water | Balance |
| Disodium EDTA | 0.05 |
| Methyl paraben | 0.15 |
| Magnesium aluminum silicate | 0.60 |
| Triethanolamine | 1.20 |
| Chloride salt of hydroxypropyltrimonium glucose | 1.00 |

| PHASE B | |
|---|---|
| Xanthan gum | 0.20 |
| Natrosol® 250HHR (ethyl cellulose) | 0.50 |
| Butylene glycol | 3.00 |
| Glycerin | 2.00 |

| PHASE C | |
|---|---|
| Sodium stearoyl lactylate | 0.10 |
| Glycerol monostearate | 1.50 |
| Stearyl alcohol | 1.50 |
| Isostearyl palmitate | 3.00 |
| Silicone fluid | 1.00 |
| Cholesterol | 0.25 |
| Sorbitan stearate | 1.00 |
| Butylated hydroxy toluene | 0.05 |
| Vitamin E acetate | 0.01 |
| PEG-100 stearate | 2.00 |
| Stearic acid | 3.00 |
| Propyl paraben | 0.10 |
| Parsol MCX® | 2.00 |
| Caprylic/capric triglyceride | 0.50 |
| Hydroxycaprylic acid | 0.01 |
| C12-15 alkyl octanoate | 3.00 |

| PHASE D | |
|---|---|
| Vitamin A palmitate | 0.10 |
| Bisabolol | 0.01 |
| Vitamin A acetate | 0.01 |
| Fragrance | 0.03 |
| Retinol 50C | 0.02 |
| Conjugated linoleic acid | 0.50 |

### EXAMPLE 2

A water-in-oil topical liquid make-up foundation according to invention is described in Table II below.

**TABLE II**

| INGREDIENT | WEIGHT % |
|---|---|
| PHASE A | |
| Cyclomethicone | 9.25 |
| Oleyl oleate | 2.00 |
| Dimethicone copolyol | 20.00 |

| PHASE B | |
|---|---|
| Talc | 3.38 |
| Pigment (iron oxides) | 10.51 |
| Spheron L-1500 (silica) | 0.50 |

| PHASE C | |
|---|---|
| Synthetic wax Durachem^{™} 0602 | 0.10 |
| Arachidyl behenate | 0.30 |

| PHASE D | |
|---|---|
| Cyclomethicone | 1.00 |
| Trihydroxystearin | 0.30 |

| PHASE E | |
|---|---|
| Laureth-7 | 0.50 |
| Propyl paraben | 0.25 |

| PHASE F | |
|---|---|
| Fragrance | 0.05 |

| PHASE G | |
|---|---|
| Water | Balance |
| Chloride salt of hydroxypropyltrimonium fructose | 3.00 |
| methyl paraben | 0.12 |
| Propylene glycol | 8.00 |
| Niacinamide | 4.00 |
| Glycerin | 3.00 |
| Sodium chloride | 2.00 |
| Sodium dehydroacetate | 0.30 |

### EXAMPLE 3

Illustrated herein is a skin cream incorporating a quat salt of the present invention.

**TABLE III**

| INGREDIENT | WEIGHT % |
|---|---|
| Glycerin | 6.93 |
| Niacinamide | 5.00 |
| Chloride salt of hydroxypropyltrimonium glucose | 5.00 |
| Permethyl^{™} 101A¹ | 3.00 |
| Sepigel^{™} 305² | 2.50 |
| Q2-1403³ | 2.00 |
| Linseed oil | 1.33 |
| Arlatone^{™} 2121⁴ | . 1.00 |
| Cetyl alcohol CO-1695 | 0.72 |
| SEFA cottonate⁵ | 0.67 |
| Tocopherol acetate | 0.50 |
| Panthenol | 0.50 |
| Stearyl alcohol | 0.48 |
| Titanium dioxide | 0.40 |
| Disodium EDTA | 0.10 |
| Glydant^{™} Plus⁶ | 0.10 |
| PEG-100 stearate | 0.10 |
| Stearic acid | 0.10 |
| Purified water | Balance |

| | |
|---|---|
| ¹ Isohexadecane (Presperse Inc., South Plainfield, NJ) ² Polyacrylamide(and)C13-14 Isoparaffin(and) Laureth-7 (Seppic Corporation, Fairfield, NJ) ³ Dimethicone(and)dimethiconol (Dow Corning Corp. Midland, MI) ⁴ Sorbitan monostearate and sucrococoate (ICI Americas Inc., Wilmington, DE) ⁵ Sucrose ester of fatty acid ⁶ DMDM hydantoin (and) iodopropynyl butylcarbamate (Lonza Inc., Fairlawn, NJ) | |

### EXAMPLE 4

Illustrative of another cosmetic composition incorporating a quat salt according to the present invention is the formula of Table IV.

**TABLE IV**

| INGREDIENT | WEIGHT % |
|---|---|
| Polysilicone-11 | 29 |
| Cyclomethicone | 59 |
| Petrolatum | 11 |
| Chloride salt of hydroxypropyltrimonium glucose | 0.2 |
| Dimethicone copolyol | 0.5 |
| Sunflowerseed oil | 0.3 |

### EXAMPLE 5

A relatively anhydrous composition incorporating a quat salt of the present invention is reported in Table V.

**TABLE V**

| INGREDIENT | WEIGHT % |
|---|---|
| Cyclomethicone | 80.65 |
| Dimethicone | 9.60 |
| Squalane | 6.00 |
| Isostearic acid | 1.90 |
| Borage seed oil | 0.90 |
| Chloride salt of hydroxypropyltrimonium glucose | 0.50 |
| Retinyl palmitate | 0.25 |
| Ceramide 6 | 0.10 |
| Tocopherol | 0.10 |

### EXAMPLE 6

An aerosol packaged foaming cleanser with a quat salt suitable for the present invention is outlined in Table VI.

**TABLE VI**

| INGREDIENT | WEIGHT % |
|---|---|
| Sunflower seed oil | 20.00 |
| Maleated soybean oil | 5.00 |
| Silicone urethane | 1.00 |
| Polyglycero-4 oleate | 1.00 |
| Sodium C14-16 olefin sulfonate | 15.00 |
| Sodium lauryl ether sulphate (25% active) | 15.00 |
| Cocoamidopropylbetaine | 15.00 |
| DC 1784® (silicone emulsion 50%) | 5.00 |
| Polyquaternium-11 | 1.00 |
| Chloride salt of hydroxypropyltrimonium glucose | 1.00 |
| Water | Balance |

### EXAMPLE 7

A disposable, single use personal care towelette product is described according to the present invention. A 70/30 polyester/rayon non-woven towelette is prepared with a weight of 1.8 grams and dimensions of 15 cm by 20 cm. Onto this towelette is impregnated a composition as outlined in Table VII below.

**TABLE VII**

| INGREDIENT | WEIGHT % |
|---|---|
| Chloride salt of hydroxypropyltrimonium glucose | 7.50 |
| glycerin | 2.00 |
| Hexylene glycol | 2.00 |
| Disodium capryl amphodiacetate | 1.00 |
| Gluconolactone | 0.90 |
| Silicone microemulsion | 0.85 |
| Witch hazel | 0.50 |
| PEG-40 hydrogenated castor oil | 0.50 |
| Fragrance | 0.20 |
| Vitamin E acetate | 0.001 |
| Water | Balance |

### EXAMPLE 8

A toilette bar illustrative of the present invention is outlined under Table VIII.

**TABLE VIII**

| INGREDIENT | WEIGHT % |
|---|---|
| Sodium soap (85/15 tallow/coconut) | 77.77 |
| Chloride salt of hydroxypropyltrimonium glucose | 3.50 |
| Glycerin | 2.50 |
| Sodium chloride | 0.77 |
| Titanium dioxide | 0.40 |
| Fragrance | 1.50 |
| Disodium EDTA | 0.02 |
| Sodium etidronate | 0.02 |
| Fluorescer | 0.024 |
| Water | Balance |

### EXAMPLE 9

A shampoo composition useful in the context of the present invention is described in Table IX below.

**TABLE IX**

| Ingredient | Weight % |
|---|---|
| Ammonium laureth sulfate | 12.00 |
| Ammonium lauryl sulfate | 2.00 |
| Cocoamidopropyl betaine | 2.00 |
| Sodium lauroamphoacetate | 2.00 |
| Chloride salt of hydroxypropyltrimonium fructose | 5.50 |
| Ethylene glycol distearate | 1.50 |
| Cocomonoethanolamide | 0.80 |
| Cetyl alcohol | 0.60 |
| Polyquaternium-10 | 0.50 |
| Dimethicone | 1.00 |
| Zinc pyridinethione | 1.00 |
| Sodium citrate | 0.40 |
| Citric acid | 0.39 |
| Sodium xylene sulfonate | 1.00 |
| Fragrance | 0.40 |
| Sodium benzoate | 0.25 |
| Kathon CG® | 0.0008 |
| Benzyl alcohol | 0.0225 |
| Water | Balance |

### EXAMPLE 10

This Example illustrates an antiperspirant/deodorant formula incorporating the moisturizing actives according to the present invention.

**TABLE X**

| Ingredient | Weight % |
|---|---|
| Cyclopentasiloxane | 44 |
| Dimethicone | 20 |
| Aluminum zirconium trichlorohydrex glycinate | 15 |
| Chloride salt of hydroxpropyltrimonium glucose | 5.0 |
| C₁₈-C₃₆ acid triglyceride | 5.0 |
| Microcrystalline wax | 3.0 |
| Glycerin | 3.0 |
| Silica | 2.5 |
| Dimethicone crosspolymer | 1.0 |
| Fragrance | 0.5 |
| Disodium EDTA | 0.4 |
| Butylated hydroxytoluene | 0.3 |
| Citric acid | 0.3 |

### EXAMPLE 11

A toothpaste according to the present invention can be formulated with the ingredients listed under Table XI.

**TABLE XI**

| Ingredients | Weight % |
|---|---|
| Zeodent 115® | 20.00 |
| Glycerin | 18.00 |
| Xanthan gum | 7.00 |
| Sodium carboxymethyl cellulose | 0.50 |
| Sodium bicarbonate | 2.50 |
| Chloride salt of hydroxypropyltrimonium fructose | 2.00 |
| Sodium laurylsulfate | 1.50 |
| Sodium fluoride | 1.10 |
| Sodium saccharin | 0.40 |
| Titanium dioxide | 1.00 |
| Pluronic F-127® | 2.00 |
| FD&C blue No. 1 | 3.30 |
| Menthol | 0.80 |
| Potassium nitrate | 5.00 |
| Water | Balance |

### EXAMPLE 12

This Example details the synthesis of glucose hydroxypropyl trimethylammonium chloride. A round bottom 250 ml flask was fitted with a mechanical stirrer. Into the flask was charged 1 M sodium hydroxide (55.5 ml, 55.5 mmol), Clearsweet^{™} 95 (10 g, 55.5 mmol) and 3-chloro-2-hydroxypropyl trimethylammonium chloride (15 ml, 55.5 mmol). Clearsweet^{™} 95 is a trademark of Cargill and contains 95.4% glucose, 2.8% maltose and 1.8% higher sugars. The 3-chloro-2-hydroxypropyl trimethylammonium chloride (CHPTMAC) was sourced from Aldrich Chemical Company as a 60% active material in water. It is also available as Quat® 188 from the Dow Chemical Company. The reactants were stirred at room temperature (∼20°C) for 18 hours. Water was removed under reduced pressure at 50°C to give a heterogeneous colorless syrup. Filtration through glass wool afforded glucose hydroxypropyl trimethylammonium chloride as a homogeneous clear and colorless syrup: m/z (ESI; M⁺ -Cl⁻) 296; HPLC (Column: YMC-ODS-AQ, S5, 120A, 4.6X250mm; Flow: 1ml/min isocratic, 100% water; Detector: RI; Temperature: 35°C) tᵣ 3.11min (Clearsweet^{™} 95 tᵣ 3.09min; CHPTMAC tᵣ 3.39min).

### EXAMPLE 13

This Example details the synthesis of fructose hydroxypropyl trimethylammonium chloride. A round bottom 250 ml flask was fitted with a mechanical stirrer. Into the flask was charged 1 M sodium hydroxide (55.5 ml, 55.5 mmol), Cornsweet^{™} (10 g, 55.5 mmol) and 3-chloro-2-hydroxypropyl trimethylammonium chloride (15 ml, 55.5 mmol). Cornsweet^{™} is obtained from the Archer Daniels Midland Corporation as a 100% fructose material. The 3-chloro-2-hydroxypropyl trimethyl ammonium chloride was sourced from Aldrich Chemical Company as a 60% active material in water. It is also available as Quat® 188 from the Dow Chemical Company. The reactants were stirred at room temperature (∼ 20°C) for 18 hours. Water was removed under reduced pressure at 50°C to give a heterogeneous colorless syrup. Filtration through glass wool afforded fructose hydroxypropyl trimethylammonium chloride as a homogeneous clear and colorless syrup: m/z (ESI; M⁺ -Cl⁻) 296; HPLC (Column: YMC-ODS-AQ, S5, 120A, 4.6X250mm; Flow: 1ml/min isocratic, 100% water; Detector: RI; Temperature: 35°C) tᵣ 3.16min (Cornsweet^{™} tᵣ 3.17min; CHPTMAC tᵣ 3.39min).

### EXAMPLE 14

Herein is described the preparation of a mixed fructose-glucose hydroxypropyl trimethylammonium chloride. A round bottom 250 ml flask was fitted with a mechanical stirrer. The flask was charged with 1M sodium hydroxide (55.5 ml, 55.5 mmol) and a mixture of Clearsweet^{™} 95 (5 g, 27.8 mmol), Cornsweet^{™} (5 g, 27.8 mmol) and 3-chloro-2-hydroxypropyl trimethylammonium chloride (15 ml, 55.5 mmol). The solution was stirred at room temperature (∼20°C) for 18 hours. Water was removed under reduced pressure at 50°C resulting in a heterogeneous light yellow syrup. Filtration through glass wool afforded a mixture of glucose hydroxypropyl trimethylammonium chloride and fructose hydroxypropyl trimethylammonium chloride as a homogeneous clear light yellow syrup: *m*/*z* (ESI; M⁺ -Cl⁻) 296; HPLC (Column: YMC-ODS-AQ, S5, 120A, 9.6X250mm; Flow: 1ml/min isocratic, 100% water; Detector: RI; Temperature: 35°C) tᵣ 3.14min (Clearsweet^{™} 95 tᵣ 3.09min; Cornsweet^{™} tᵣ 3.17min. CHPTMAC tᵣ 3.39min).

### EXAMPLE 15

This Example provides the results of moisturization efficacy tests. These tests involved evaluation on porcine epidermis utilized as a human skin model. The equipment and protocol are outlined below.

An environmental microbalance (Model MB-300W, VTI Corp., 2708 W 84^{th} Street, Hialeah, FL 33016) was programmed to measure the change in weight of porcine skin as a function of relative humidity at a constant temperature and air flow. The porcine skin was evaluated before and after treatment with aqueous solutions of humectants to determine the adsorption and retention of moisture.

Sample preparation was done as follows:
1. Epidermal sections of porcine skin were cut to approximately 4 cm x 1 cm.
2. The skin was washed in a 10% detergent solution and dried in a dessicator to a constant weight. This represents the untreated material.
3. The skin was soaked in a 1% by weight aqueous solution of the test sample for 15 minutes, excess fluid was blotted off and the skin was dried to constant weight in a dessicator. This represents the treated material.

The sequence of conditions for the microbalance was as follows:
1. 30 minutes at 0% relative humidity (ensures that sample is dry).
2. 90 minutes at 80% relative humidity (determines amount of water picked up).
3. 90 minutes at 20% relative humidity (determines amount of water retained).

The experiments were conducted as follows:
1. The weight of a piece of untreated skin was recorded continuously during the sequence.
2. The piece of untreated skin was treated with the test sample.
3. The weight of the treated piece of skin was recorded continuously during the sequence.
4. Data reduction consisted of calculating the percent weight change from the initial weight for the untreated and treated pieces of skin.
5. The reported data was the difference between each treated piece and its corresponding untreated piece. Results are recorded in Table XII.

**TABLE XII**

| Sample* | From 0 through 80% Relative Humidity | From 0 to 80 to 20% Relative Humidity |
|---|---|---|
| Mixed fructose/glucose monoquat salt | 2.50 | 4.445 |
| (Example 14) | | |
| Honeyquat® | 0.31 | 0.70 |
| Mixed fructose/glucose triquat salt | 0.26 | 0.16 |
| Honey | 0.24 | 0.02 |
| Quat® 188 | 0.21 | 0.10 |
| Glycerin | 0.21 | 0.10 |

| | | |
|---|---|---|
| * All samples tested at 1% active material in water solution. Data points represent the difference in weight of treated skin minus untreated skin. | | |

Evident from the results is that the mono-quaternary amonium functionalized (monoquat) fructose/glucose was not only effective for moisturizing at relatively high humidity but also exceptional at relatively low humidity. This contrasts with a tri-quaternary functionalized (triquat) fructose/glucose mixture which hardly showed any moisturization activity. These results were especially significant relative to glycerin which is normally used for moisturization purposes in cosmetic formulations.

## Claims

1. A personal care composition comprising:
(i) from 0.1 to 30% by weight of a quaternary ammonium compound which is a salt of hydroxypropyltri(C₁-C₃ alkyl)ammonium mono-substituted monosaccharide; and
(ii)a cosmetically acceptable carrier.

2. The composition according to claim 1 wherein the monosaccharide is selected from the group consisting of ribose, deoxyribose, glucose, fructose, arabinose, xylose, lyxose, allose, altrose, gulose, mannose, idose, galactose and talose.

3. The composition according to claim 2 wherein the salt is formed from glucose or fructose as monosaccharide.

4. The composition according to any one of claims 1 to 3 wherein the salt is present in an amount from 1.5 to 12% by weight of the composition.

5. The composition according to any one of the preceding claims wherein the salt is selected from structures I and II: 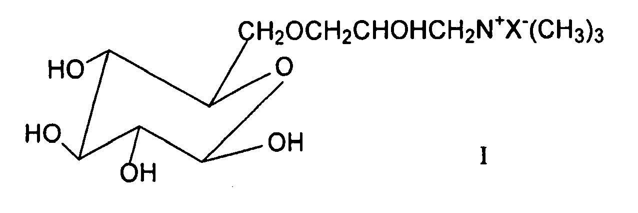 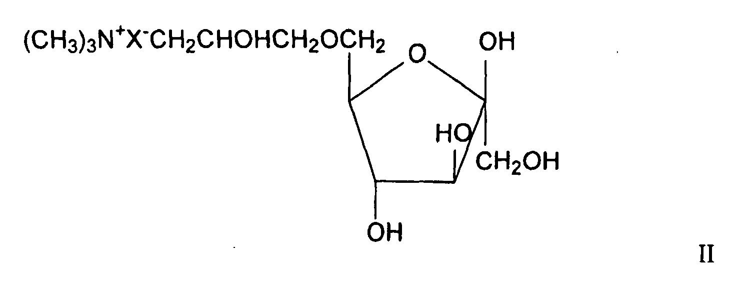 wherein X ⁻ is a cosmetically acceptable organic or inorganic counterion.

6. The composition according to claim 5 wherein the salt is a chloride of hydroxypropyltrimonium glucose or hydroxypropyltrimonium fructose or combinations thereof.

7. The composition according to any one of the preceding claims further comprising by weight of the quaternary ammonium compound from 0 to 20% of monosaccharide which is di- or tri- substituted with hydroxypropyl (C₁-C₃ alkyl) ammonium groups.

8. The composition according to any one of the preceding claims which is selected from the group consisting of leave-on skin lotions and creams, shampoos, hair conditioners, shower gels, toilette bars, antiperspirants, deodorants, dental products, shave creams, depilatories, lipsticks, foundations, mascara, sunless tanner and sunscreen lotions.

9. A method for improving skin moisturization comprising applying to skin a personal care composition according to any one of the preceding claims.

10. Use of a salt of hydroxypropyltri(C₁-C₃ alkyl)ammonium mono-substituted monosaccharide as a moisturiser.

11. The use according to claim 10 wherein the salt is a chloride of hydroxypropyltrimonium glucose or hydroxypropyltrimonium fructose or combinations thereof.

## Patentansprüche

1. Körperpflegezusammensetzung, umfassend
(i) 0,1 bis 30 Gewichtsprozent einer quaternären Ammoniumverbindung, die ein Salz von Hydroxypropyltri(C₁-C₃-alkyl)ammonium-monosubstituiertem Monosaccharid darstellt; und
(ii) einen kosmetisch verträglichen Träger.

2. Zusammensetzung nach Anspruch 1, worin das Monosaccharid aus der Gruppe, bestehend aus Ribose, Desoxyribose, Glucose, Fructose, Arabinose, Xylose, Lyxose, Allose, Altrose, Gulose, Mannose, Idose, Galactose und Talose, ausgewählt ist.

3. Zusammensetzung nach Anspruch 2, worin das Salz aus Glucose oder Fructose als Monosaccharid gebildet wird.

4. Zusammensetzung nach einem der Ansprüche 1 bis 3, worin das Salz in einer Menge von 1,5 bis 12 Gewichtsprozent der Zusammensetzung vorliegt.

5. Zusammensetzung nach einem der vorangehenden Ansprüche, worin das Salz aus Strukturen I und II ausgewählt ist: 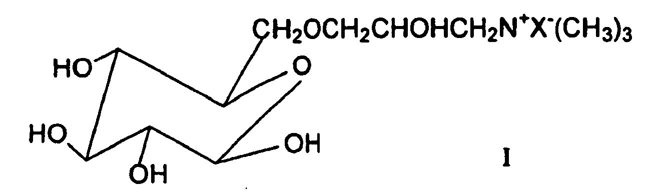 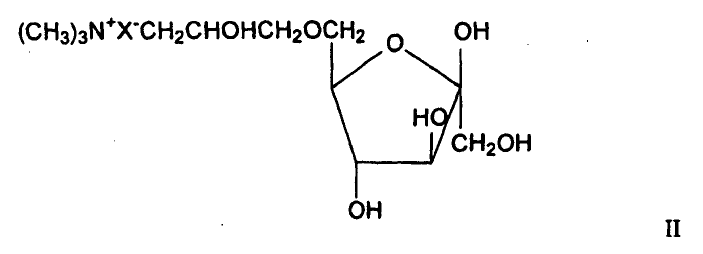 worin X⁻ ein kosmetisch verträgliches organisches oder anorganisches Gegenion darstellt.

6. Zusammensetzung nach Anspruch 5, worin das Salz ein Chlorid von Hydroxypropyltrimoniumglucose oder Hydroxypropyltrimoniumfructose oder Kombinationen davon darstellt.

7. Zusammensetzung nach einem der vorangehenden Ansprüche, weiterhin umfassend, auf das Gewicht der quaternären Ammoniumverbindung, 0 bis 20 % Monosaccharid, das mit Hydroxypropyl(C₁-C₃-alkyl)ammonium-Gruppen di- oder tri-substituiert ist.

8. Zusammensetzung nach einem der vorangehenden Ansprüche, die aus der Gruppe, bestehend aus Leave-on-Hautlotionen und -Cremes, Shampoos, Haarkonditionierern, Duschgelen, Toilettenriegeln, schweißhemmenden Mitteln, Deodorantien, Zahnprodukten, Rasiercremes, Enthaarungsmitteln, Lippenstiften, Grundierungen, Mascara, sonnenlosen Bräunungsmitteln und Sonnenschutzlotionen, ausgewählt ist.

9. Verfahren zum Verbessern der Hautbefeuchtung, umfassend Auftragen einer Körperpflegezusammensetzung gemäß einem der vorangehenden Ansprüche auf die Haut.

10. Verwendung eines Salzes von Hydroxypropyltri(C₁-C₃-alkyl)-ammonium-monosubstituiertem Monosaccharid als ein Befeuchtungsmittel.

11. Verwendung nach Anspruch 10, worin das Salz ein Chlorid von Hydroxypropyltrimoniumglucose oder Hydroxypropyltrimoniumfructose oder Kombinationen davon darstellt.

## Revendications

1. Composition de produit de soins personnels comprenant :
(i) de 0,1 % à 30 % en poids d'un composé d'ammonium quaternaire qui est un sel de monosaccharide d'hydroxypropyltri(alkyle en C₁-C₃) ammonium monosubstitué ; et
(ii) un véhicule cosmétiquement acceptable.

2. Composition selon la revendication 1 dans laquelle le monosaccharide est choisi dans le groupe comprenant le ribose, le désoxyribose, le glucose, le fructose, l'arabinose, le xylose, le lyxose, l'allose, l'altrose, le gulose, le mannose, l'idose, le galactose et le talose.

3. Composition selon la revendication 2 dans laquelle le sel est constitué à partir du glucose ou du fructose sous la forme d'un monosaccharide.

4. Composition selon l'une quelconque des revendications 1 à 3 dans laquelle le sel est présent en une quantité de 1,5 % à 12 % en poids de la composition.

5. Composition selon l'une quelconque des revendications précédentes dans laquelle le sel est choisi parmi les structures I et II 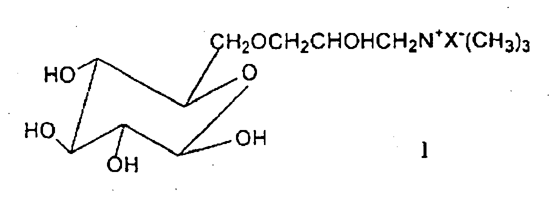 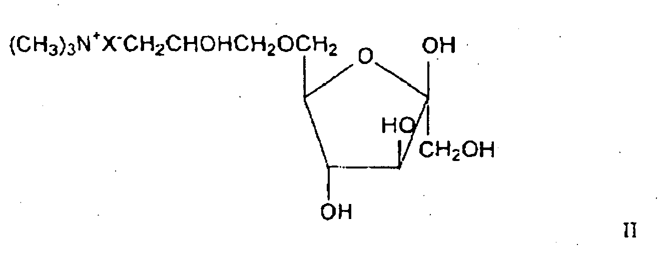 dans lesquelles X⁻ est un contre-ion organique ou inorganique cosmétiquement acceptable.

6. Composition selon la revendication 5 dans laquelle le sel est un chlorure d'hydroxypropyltrimonium glucose ou d'hydroxypropyltrimonium fructose ou des combinaisons de ceux-ci.

7. Composition selon l'une quelconque des revendications précédentes comprenant en outre en poids du composé d'ammonium quaternaire de 0 % à 20 % de monosaccharide qui est di- ou tri-substitué par des groupes hydroxypropyl (alkyle en C₁-C₃) ammonium.

8. Composition selon l'une quelconque des revendications précédentes qui est choisie dans le groupe comprenant des lotions et des crèmes pour lesquels il n'est pas prévu de rinçage, des shampoings, des après-shampoings, des gels douche, des savonnettes de toilette, des antitranspirants, des déodorants, des produits dentaires, des crèmes de rasage, des dépilatoires, des rouges à lèvres, des fonds de teint, des mascaras, des auto-bronzants et des lotions écran solaire.

9. Procédé destiné à améliorer l'hydratation de la peau comprenant l'application sur la peau d'une composition de produit de soins personnels selon l'une quelconque des revendications précédentes.

10. Utilisation d'un sel de monosaccharide d'hydroxypropyltri(alkyle en C₁-C₃) ammonium monosubstitué en tant qu'hydratant.

11. Utilisation selon la revendication 10 dans laquelle le sel est un chlorure d'hydroxypropyltrimonium glucose ou d'hydroxypropyltrimonium fructose ou des combinaisons de ceux-ci.
